Europäisches Patentamt

European Patent Office

Office européen des brevets

(11) Publication number: **0 208 799**
**B1**

# EUROPEAN PATENT SPECIFICATION

(45) Date of publication of the patent specification:
26.09.90

(51) Int. Cl.⁵: **A61K 7/42**

(21) Application number: **85108832.8**

(22) Date of filing: **15.07.85**

(54) Whitening cosmetic composition.

(43) Date of publication of application:
**21.01.87 Bulletin 87/4**

(45) Publication of the grant of the patent:
**26.09.90 Bulletin 90/39**

(84) Designated Contracting States:
**DE FR GB IT**

(56) References cited:
**GB-A- 2 052 973**

**CHEMICAL ABSTRACTS, vol. 96, no. 16, April 1982, page 447, no. 129592f, Columbus, Ohio, US**

(73) Proprietor: **Sansho Seiyaku Co., Ltd., 26-7, Oike 2-chome, Ohnojo-shi Fukuoka-ken(JP)**

(72) Inventor: **Higa, Yoshitaka, 754-2, Kokubu, Dazaifu-shi Fukuoka-ken(JP)**

(74) Representative: **Patentanwälte Müller-Boré, Deufel, Schön, Hertel, Lewald, Otto, Postfach 26 02 47 Isartorplatz 6, D-8000 München 26(DE)**

**Description**

The present invention relates to a synergistic skin whitening cosmetic composition.

For removing stains, freckles, etc. appearing on the skin sand exerting a whitening effect on the same a great variety of cosmetic compositions is known. These compositions contain peroxides such as hydrogen peroxide, zinc peroxide, magnesium peroxide, sodium peroxide, zinc perborate, etc. However, compositions containing these peroxides because of the instability of the same are difficultly to store, and, additonally, their withening effect is unsatisfactory.

Cosmetic compositions containing vitamin C, cystein, colloidal sulfur, etc. have also been formulated, however, their whitening effect is also unsatisfactory.

Withening compositions containing kojic acid obtained by cultivation of funghi such as koji moulds are known from JP-A 18569/81 and 3538/78 and GB-A 2 052 973.

Whitening compositions containing derivatives of kojic acid are disclosed in JP-A 7710/81, 7776/81 and 79616/81.

Cosmetics containing pyrone compounds, chromone compounds or flavonol compounds are known for example from JP-A 3538/78, 92305/80, 111410/80, 11141/80, 143908/80, 157580/80, 14517/82, 35506/82, 131911/93, etc.

Cosmetic compositions containing placenta extracts are known from JP-A 15399/60 and Chemical Abstracts, No. 129, 529f Vol. 96 (10/82).

The aim of the present invention is to provide whitening cosmetic compositions which, in comparison to the known compositions, show an improved whitening effect.

The present invention is concerned with a synergistic skin whitening cosmetic composition comprising a placenta extract and kojic acid or a kojic acid derivative selected from monofatty acid ester of kojic acid, difatty acid ester of kojic acid, kojic acid monocinnamoate and kojic acid monobenzoate.

The whitening cosmetic compositions of the present invention have an extremely improved whitening effect which – as the following example and comparative example show – is much better than the whitening effect of compositions containing kojic acid alone and placenta extract alone.

The placenta extracts which are employed in the compositions of the present invention are aqueous solutions obtained by extracting water-soluble placenta components with water, i.e. by washing human placenta or placenta of animals such as cows with water, withdrawing the blood, crushing, freezing, and removing impurities from the extract. Extracts obtained in this way are commercially available as raw materials for cosmetics. According to the present invention human-derived placenta extracts are preferred.

Examples of monofatty esters of kojic acid are kojic acid monopalmitate, kojic acid monobutyrate, kojic acid monocaprylate and kojic acid monostearate (see for example JP-A 77272/81). Examples of difatty acid ester of kojic acid are kojic acid dipalmitate, kojic acid dibutyrate, kojic acid dioleate and kojic acid distearate (see for example JP-A 7776/81). Kojic monicinnamoate and kojic acid monobenzoate are for example disclosed in JP-A 33207/84.

In the whitening cosmetic compositions of the present invention the placenta extract is used an amount of approximately 0,1 to 10% by weight, based on the total composition, and the kojic acid or kojic acid derivatives are used in an amount of approximately 0,1 to 3% by weight, also based on the total amount of the compositions, to achieve a sufficient whitening effect.

The whitening cosmetic compositions of the present invention are applicable to the skin as lotions, creams, emulsions, packs, etc. These formulations are prepared by incorporating the placenta extract and the kojic acid and/or kojic acid derivative(s) into cosmetic bases, aids, etc. conventionally used for preparing cosmetic compositions.

For example a process for producing lotions comprises dissolving moisturizing agents, skin nutrients such as glycerol, propylene glycol, etc., in purified water on the one hand, and dissolving preservatives, aromatics, ect. in alcohols on the other hand, mixing both solutions and solubilizing them at room temperature, and incorporating placenta extract and kojic acid or kojic acid derivative(s) into the water-soluble portions.

A process for producing creams comprises incorporating hydrophilic components, for example moisturizing agents, like glycerol, sorbitol, etc., into purified water to prepare an aqueous phase portion, incorporating oily componentens such as preservatives, surface active agents, etc. into solid oils, such as beeswax, paraffin, microcrystalline wax, ceresine, higher fatty acids and hardened oils, semi-solid oils, such as vaseline, lanoline, and glycerides, liquid oils, such as squalane, liquid paraffin and various ester oils, to produce an oily phase portion, warming the thus obtained aqueous phase portion and gradually adding the oily phase portion warmed to the same temperature to this aqueous phase portion under mild stirring to emulsify the mixture and to incorporate the active ingredient into the aqueous phase portion. By this way creams are obtained.

A process for preparing emulsions comprises incroporating moisturing agents, such as glycerol, or pH-controlling agents, such as acids or alkalis, into purified water, mixing under heating, and adding ethanol to the mixture to prepare an aqueous phase portion. On the other hand oily components, such as preservatives, surface active agents, etc., are incorporated into solid oils, such as beeswax, paraffin, etc., semi-solid oils, such as vaseline, lanoline, etc., liquid oils, such as squalane, liquid paraffin, various ester oils, etc., and mixing under heating to prepare an oily phase portion. Then the oily phase portion is added to the aqueous phase portion to obtain a preliminary emulsion, whereafter protective colloids, such as carbovinyl polymers, carboxymethyl cellulose, etc., are added to the emulsion and are homogeneously emulsified by a homomixer to make an emulsion. Then placenta extract and kojic acid or kojic acid derivative(s) are added and incorporated into the aqueous phase protion. In this way an emulsion is obtained.

Facial packs for example can be prepared by incorporating moisturing agents, like glycerol, etc., film forming agents, like polyvinylalcohol, VEE GUM® etc. into purified water, allowing to swell these components, adding powders, like kaolin, talc and zinc oxide, and, if necessary, adding ethanol having dissolved therein flavoring agents, preservatives, etc., kneading the mixture to a paste form, and adding the placenta extract in kojic acid or kojic acid derivative(s) to be incorporated into the aqueous phase portion to make facial packs.

The excellent whitening effect of the whitening cosmetic compositions of the present invention is based on a surprising synergistic effect of placenta extract and kojic acid or kojic acid derivative(s).

This effect is demonstrated by decolorisation test using incubated chromocytes.

Test Example

1. Cells used
Mouse melanoma B 16 cells (called B 16 cells)
2. Condition for incubation
Using Eagle's MEM medium supplemented with 10% bovine fetal serum, 2,2 g/l of hydrogen sodium carbonate and 5 mM N-2-hydroxyethylpiperazine-N'-ethanesulfonic acid, incubation was performed in a $CO_2$ incubator set at 37°C in 5% $CO_2$.
3. Test fractions
Fraction A: fraction to which neither kojic acid nor the placenta extract was added.
Fraction B: fraction to which kojic acid was added;
0.1 mM was added to Fraction B–I;
0.5 mM was added to Fraction B–II;
1.0 mM was added to Fraction B–III;
2.0 mM was added to Fraction B–IV;
3.0 mM was added to Fraction B–V;
Fraction C: fraction to which the placenta extract was added; after freeze-drying 100 ml of the placenta extract, this was dissolved in 100 ml of a cell culture solution; the thus obtained solution was used as a raw solution.
1/16 volume of the raw solution was added to Fraction C–I;
1/8 volume of the raw solution was added to Fraction C–II;
1/4 volume of the raw solution was added to Fraction C–III;
1/2 volume of the raw solution was added to Fraction C–IV;
the raw solution was added to Fraction C–V.
Fraction D: fraction to which kojic acid and the placenta extract were added in combination;
0.1 mM of kojic acid and 1/16 volume of the raw solution of the placenta extract were used in combination as Fraction D–I;
0,5 mM of kojic acid and 1/8 volume of the raw solution of the placenta extract were used in combination as Fraction D–II;
1.0 mM of kojic acid and 1/4 volume of the raw solution of the placenta extract were used in combination as Fraction D–III;
2.6 mM of kojic acid and 1/2 volume of the raw solution of the placenta extract were used in combination as Fraction D–IV;
3.0 mM of kojic acid and the raw solution of the placenta extract were used in combination as Fraction D–V;
4. Evaluation of inhibitory action against formation of melanin
On each test fraction $1.2 \times 10^5$ cells/dish of B 16 cells were inoculated. After incubation for 6 days, proliferated cells were dispersed with trypsin and then collected by centrifugal separation at 1000 r.p.m. × 5 minutes. The blackening degree was evaluated with the naked eye.
−: The blackening degree was almost the same as that of the fraction to which nothing was added.
±: The blackening degree was somewhat less than that of the fraction to which nothing was added.
+: The blackening degree was obviously less than that of the fraction to which nothing was added.
++: The blackening degree was slightly noted.
+++: The color was white to gray but no blackening was noted.
5. Results of evaluation
The results are shown in the table below.

| Fraction | I | II | III | IV | V |
|----------|---|----|----|----|----|
| A | − | − | − | − | − |
| B | − | − | + | + | ++ |
| C | − | − | ± | + | ++ |
| D | + | ++ | +++ | +++ | +++ |

As is evident from the above results, the effective components of the present invention provide a marked inhibitory effect against the formation of melanin, not only as compared to the fraction to which nothing was added but also as compared to the fraction to which one of the effective components of the present invention was added alone.

As already mentioned it is known that kojic acid or kojic acid derivatives each are capable of inhibiting the formation of melanin. As a result of the analysis of the mechanism of inhibition the formation of melanin in cell lines using B 16 cells the present inventors could prove that kojic acid forms a chelate with copper ions which affect the activity of tyrosinase which takes part in the formation of melanin on the initial stage thereby to prevent tyrosinase activity and inhibit the formation of melanin. Placenta extract, on the other hand, does not chelate the copper ions, and the mechanism of the placenta extract on preventing the formation of melanin is differing from that of kojic acid. Therefore, the synergistic effect of both kojic acid or kojic acid derivative(s) and the placenta extract is believed to be based on different action of inhibiting the formation of melanin.

The following two examples illustrate the present invention.

Example 1

(Cream)
Kojic acid 1.0%
Placenta extract 5.0%
Polyoxyethylene stearyl ether 2.09%
Polyoxyethylene cetyl ether 2.91%
Beeswax 4.0%
Cetanol 3.0%
Isopropyl palmitate 2.0%
Liquid paraffin 15.0%
Polyethylene glycol monostearate 0.5%
Purified water 64.4%
Methyl p-oxybenzoate 0.1%

Example 2

(Emulsion)
Kojic acid 2.0%
Placenta extract 4.0%
Self-emulsified type glycerol monostearate 1.11%
Polyoxyethylene cetyl ether 1.89%
MC stearic acid 2.0%
Cetanol 1.0%
Isopropyl myristate 2.0%
p-Oxybenzoic acid 0.1%
Aromatics trace

Example 3

(Facial Pack)
Kojic acid 0.5%
Placenta extract 6.0%
Ethanol 3.0%
Methyl p-oxybenzoate 0.1%
Carboxyvinyl polymer 1.0%
Calcium carbonate 0.3%
Titanium dioxide 0.02%
Purified water 89.08%
Aromatics trace

Example 4

(Lotion)
Kojic acid 3.0%
Placenta extract 10.0%
Ethanol 10.0%
Methyl p-oxybenzoate 0.1%
Citric acid 0.2%
Aromatics trace
Purified water 76.7%

**Claims**

1. A syneregistic skin whitening cosmetic composition comprising a placenta extract and kojic acid or a kojic acid derivative selected from monofatty acid ester of kojic acid, difatty acid ester of kojic acid, kojic acid monocinnamoate and kojic acid monobenzoate.

2. A syneregistic skin whitening cosmetic composition according to claim 1 wherein said placenta extract is a human placenta extract.

**Patentansprüche**

1. Synergistische, die Haut weichmachende kosmetische Zubereitung aus einem Placentaextrakt und Kojisäure oder einem Kojisäurederivat, ausgewählt aus einem Monofettsäureester von Kojisäure, Difettsäureester von Kojisäure, Kojisäuremonocinnamoat und Kojisäuremonobenzoat.

2. Synergistische, die Haut weichmachende kosmetische Zubereitung nach Anspruch 1, wobei der Placentaextrakt ein menschlicher Placentaextrakt ist.

**Revendications**

1. Composition cosmétique synergique convenant pour éclaircir ou éliminer les taches de la peau comprenant un extrait de placenta et de l'acide kojique ou un dérive d'acide kojique choisi parmi les monoesters d'acides gras de l'acide kojique, les diesters d'acides gras de l'acide kojique, le monocinnamate d'acide kojique et le monobenzoate d'acide kojique.

2. Composition cosmétique synergique convenant pour éclaircir ou éliminer les taches de la peau selon la revendication 1 dans laquelle l'extrait de placenta est un extrait de placenta humain.